(19) Europäisches Patentamt
European Patent Office
Office européen des brevets

(11) Publication number: 0 195 496 A2

(12) **EUROPEAN PATENT APPLICATION**

(21) Application number: 86300387.7

(22) Date of filing: 21.01.86

(51) Int. Cl.[4]: A 61 K 31/52

(30) Priority: 21.01.85 GB 8501488

(43) Date of publication of application: 24.09.86 Bulletin 86/39

(84) Designated Contracting States: BE CH DE FR GB IT LI LU NL SE

(71) Applicant: BEECHAM GROUP PLC
Beecham House Great West Road
Brentford Middlesex TW8 9BD(GB)

(72) Inventor: Arch, Jonathan Robert Sanders
38 Warren Road
Banstead Surrey SM7 1LA(GB)

(74) Representative: Blake, John Henry Francis et al,
Beecham Pharmaceuticals Great Burgh Yew Tree
Bottom Road
Epsom, Surrey KT18 5XQ(GB)

(54) **Use of xanthines in the treatment of proliferative skin disease.**

(57) 1. A method for the treatment of proliferative skin disease in human or non-human mammals which comprises administering to the human or non-human mammal in need of such treatment an effective non-toxic amount of a compound of formula (I):

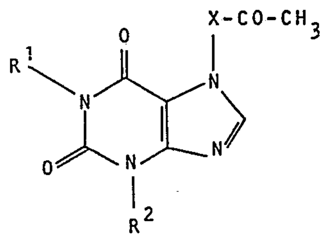

(I)

wherein $R^1$ and $R^2$, which may be the same or different, each represents a straight-chain or branched-chain alkylradical of 2 to 6 carbon atoms, or a cyclohexyl, alkoxyalkyl or hydroxyalkyl radical, and X represents a hydrocarbon radical having up to 4 carbon atoms which may be substituted by a methyl group;

a compound of formula (II):

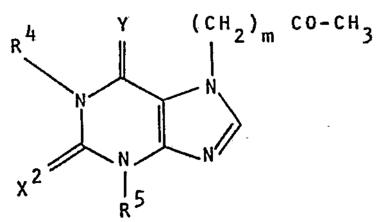

(II)

wherein

$X^2$ is sulphur and Y is oxygen or sulphur:

$R^4$ is an alkyl group of up to 6 carbon atoms;

$R^5$ is an alkyl group of up to 6 carbon atoms; and m is 1;

or

$X^2$ is oxygen and Y is sulphur; one of $R^4$ and $R^5$ is an alkyl group of up to 6 carbon atoms and the other is an alkyl group of 2 to 3 carbon atoms; and m is 1 or 2;

a compound of formula (III):

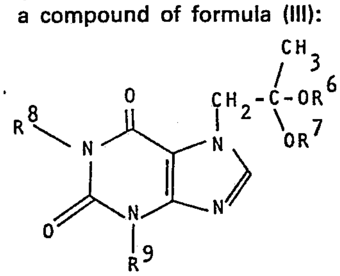

(III)

./...

EP 0 195 496 A2

wherein

$R^6$ is a lower alkyl group and $R^7$ is a lower alkyl group; or $R^6$ is linked to $R^7$ so that the $OR^6$ and $OR^7$ moieties and the carbon atom to which they are attached form a 1,3-dioxacyclohexa -2,2-diyl, 1,3-dioxacyclopenta -2,2-diyl or 1,3-dioxacyclohepta-2,2-diyl diradiacal; and $R^8$ and $R^9$ are the same or different and are each a lower alkyl group:

a compound of formula (IV):

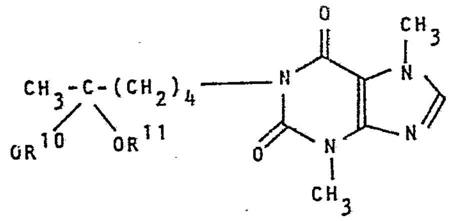

(IV)

wherein $R^{10}$ is a lower alkyl group and $R^{11}$ is a lower alkyl group optionally linked to $R^{10}$ so that the $OR^{10}$ and $OR^{11}$ moieties and the carbon atom to which they are attached form a 1,3-dioxacyclohexa-2,2-diyl, 1,3-dioxacyclo-penta-2,2-diyl or 1,3-dioxacyclohepta-2,2-diyl diradical; or

a compound of formula (V):

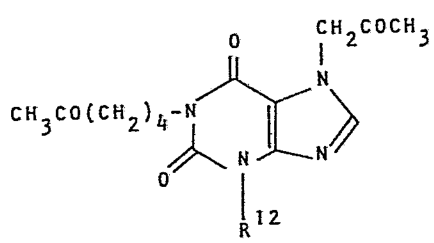

(V)

wherein $R^{12}$ is $C_{1-4}$ alkyl.

TITLE MODIFIED see front page

NOVEL TREATMENT

The present invention relates to a method for the treatment of proliferative skin diseases such as psoriasis and compounds for use in the method.

As used herein the expression 'proliferative skin diseases' means benign and malignant proliferative skin diseases which are characterized by accelerated cell division in the epidermis, dermis or appendages thereto, associated with incomplete tissue differentiation. Such diseases include: psoriasis, atopic dermatitis, non-specific dermatitis, primary irritant contact dermatitis, allergic contact dermatitis, basal and squamous cell carcinomas of the skin, lamellar ichthyosis, epidermolytic hyperkeratosis, premalignant sun induced keratosis, non-malignant keratosis, acne, and seborrheic dermatitis in humans and atopic dermatitis and mange in domesticated animals.

British Patent Specification No.1441562 describes compounds of formula (I):

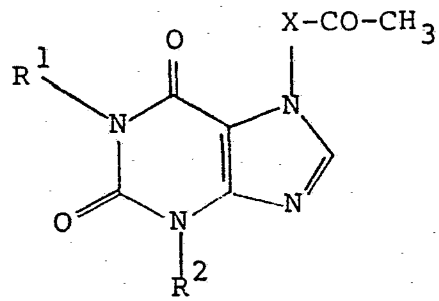

(I)

wherein $R^1$ and $R^2$, which may be the same or different, each represents a straight-chain or branched-chain alkylradical of 2 to 6 carbon atoms, or a cyclohexyl,

alkoxyalkyl or hydroxyalkyl radical, and X represents a hydrocarbon radical having up to 4 carbon atoms which may be substituted by a methyl group. The compounds of the formula (I) are described as effective in increasing the blood flow through skeletal muscles at the same time showing low toxicity.

British Patent Specification No 1,496,315 discloses compounds of formula (I) wherein $R^1$ is n-butyl, $R^2$ is ethyl or n-propyl and X is a straight chain alkylene group of 1 to 4 carbon atoms optionally substituted by a methyl group.

European Patent Specification No 5015 discloses that the compound of formula (I) wherein $R^1$ and $R^2$ each represent $R^3$, wherein $R^3$ is an n-butyl group and X is a $CH_2$ group, i.e.1,3-di-n-butyl-7-oxopropyl xanthine, is effective in increasing oxygen tension and contractility in ischaemic skeletal muscle and is therefore of potential use in the treatment of peripheral vascular disease.

European Patent Specification No 0018136 discloses compounds of formula (II)

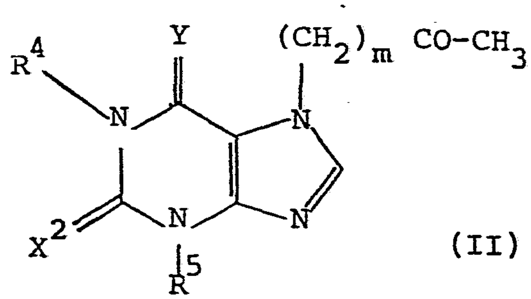

(II)

wherein,

$X^2$ is sulphur and Y is oxygen or sulphur:

$R^4$ is an alkyl group of up to 6 carbon atoms;

$R^5$ is an alkyl group of up to 6 carbon atoms; and

m is 1; or

$X^2$ is oxygen and Y is sulphur; one of $R^4$ and $R^5$ is an alkyl group of up to 6 carbon atoms and the other is an alkyl group of 2 to 3 carbon atoms; and m is 1 or 2.

The compounds of formula (II) are disclosed as effective in improving the metabolic status of ischaemic skeletal muscle by increasing oxygen tension and/or contractility in the tissue and are thus of potential use as agents for the treatment of peripheral vascular disease such as intermittent claudication.

European Patent Specification No 0018135 discloses compounds of formula (III):

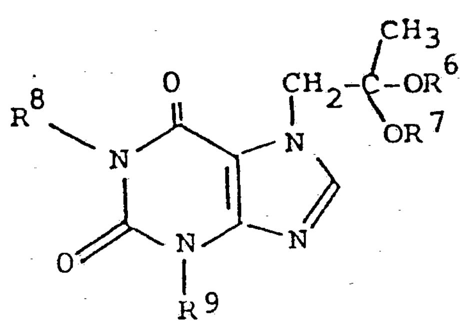

(III)

wherein

$R^6$ is a lower alkyl group and $R^7$ is a lower alkyl group; or $R^6$ is linked to $R^7$ so that the $OR^6$ and $OR^7$ moieties and the carbon atom to which they are attached form a 1,3-dioxacyclohexa -2,2-diyl, 1,3-dioxacyclopenta -2,2-diyl or 1,3-dioxacyclohepta-2,2-diyl diradical; and $R^8$ and $R^9$ are the same or different and are each a lower alkyl group; in which the term ''lower'' means containing 1 to 4 carbon atoms.

The compounds of formula (III) are described for use in the treatment of vascular disorders such as intermittent claudication.

European Patent Specification No 0017478 discloses pharmaceutical compositions containing compounds of formula (IV):

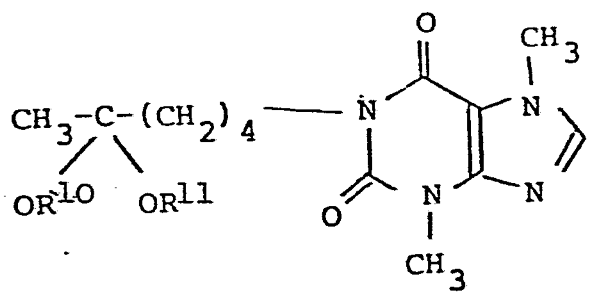

(IV)

wherein $R^{10}$ is a $C_{1-4}$ alkyl group and $R^{11}$ is a $C_{1-4}$ alkyl group optionally linked to $R^{10}$ so that the $OR^{10}$ and $OR^{11}$ moieties and the carbon atom to which they are attached form a 1,3-dioxacyclohexa-2,2-diyl, 1,3-di oxacyclopenta-2,2-diyl or 1,3-dioxacyclohepta-2,2-diyl diradical.

The compositions containing the compounds of formula (IV) are described as being useful in promoting blood flow through skeletal muscles which is desirable for the treatment of intermittent claudication.

European Published Application No 0042706 discloses compounds of formula (V);

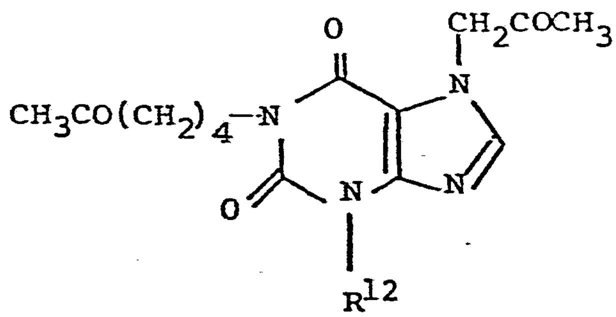

(V)

wherein $R^{12}$ is $C_{1-4}$ alkyl. The compounds of formula (V) are described as active in increasing oxygen tension in ischaemic skeletal muscle and as useful in the treatment of peripheral vascular disorders such as intermittent claudication.

It has now been discovered that compounds of formula (I), (II), (III), (IV) or (V), as hereinbefore defined, act as phosphodiesterase inhibitors and elevate cyclic AMP levels and are therefore of potential use in the treatment of proliferative skin disease in human or non-human mammals.

Accordingly, the present invention provides a method for the treatment of proliferative skin disease in human or non-human mammals which comprises administering to the human or non-human mammal in need of such treatment an effective amount of a compound of formula (I), (II), (III), (IV) or (V) as hereinbefore defined.

The present invention also provides a compound of formula (I), (II), (III), (IV) or (V) for use in the treatment of proliferative skin disease.

In a further aspect of the present invention there is provided the use of a compound of formula (I), (II), (III), (IV) or (V) for the manufacture of a medicament for treating proliferative skin disease in human or non-human mammals.

In a further aspect of the present invention there is provided a pharmaceutical composition for use in the treatment of proliferative skin disease in human or non-human mammals, which comprises a compound of formula (I), (II), (III), (IV) or (V) and a pharmaceutically acceptable carrier therefor.

The administration to the human or non-human mammal may be by way of oral administration, parenteral or topical administration.

Suitable compounds of formula (I) are those exemplified in British Patent specification Nos. 1,441,562, and 1,496,315 and European Patent No 5015.

Suitably $R^1$ is ethyl, propyl or butyl
Suitably $R^2$ is ethyl, propyl or butyl
Suitably X is $-CH_2-$, $-(CH_2)_2-$, $-(CH_2)_3-$, $-CH(CH_3)-$ or $-CH(CH_3)CH_2-$

Favourably, X is $-CH_2-$, $-(CH_2)_2-$ or $-(CH_2)_4-$.

Preferably, $R^1$ is n-butyl.

Preferably, $R^1$ is iso-propyl.

Preferably, $R^2$ is n-butyl.

Preferably, $R^2$ is n-propyl or iso-propyl.

Preferably, X is $-CH_2-$.

A preferred compound of formula (I) is that selected from the list consisting of:

1-n-butyl 3-ethyl-7-(3-oxobutyl) xanthine;

1,3- di-iso-propyl-7-(2-oxopropyl) xanthine;

1-n-butyl 3-n-propyl-7-(2-oxopropyl) xanthine;

1,3- di-n-butyl-7-(2-oxopropyl) xanthine;

1,3-di-n-butyl-7-(3-oxobutyl) xanthine;

1,3-di-n-butyl-7-(5-oxo hexyl)xanthine;
and 1-n-butyl-3-ethyl-7-(3-oxobutyl)xanthine.

A particularly preferred compound of formula (I) is 1,3-di-n-butyl-7-(2-oxopropyl) xanthine.

A particularly preferred compound of formula (I) is 1-n-butyl-3-n-propyl-7-(2-oxopropyl) xanthine.

Suitable compounds of formula (II) are those exemplified in European Patent Specification No 0018136.

Suitably, $R_4$ is ethyl, propyl or butyl.
Suitably, $R_5$ is ethyl, propyl or butyl.
Suitably, $X^2$ is sulphur and Y is oxygen.
Suitably, $X^2$ is oxygen and Y is sulphur.

Suitably, m is 1.

Preferably, $R^4$ is n-butyl
Favourably, $R^5$ is ethyl or n-butyl, preferably n-butyl.
Preferably, $X^2$ is sulphur and Y is oxygen.

A preferred compound of formula (II) is 1,3-di-n-butyl-7-(2-oxopropyl)-2-thioxanthine.

Suitable compound of formula (III) are those exemplified in European Patent Specification No. 0018135.

Suitably, $R^6$ is methyl. Suitably $R^7$ is methyl. Suitably, $R^6$ is linked to $R^7$ so that the $OR^6$ and $OR^7$ moieties and the carbon atom to which they are attached form a 1,3-dioxacyclopenta-2,2-diyl or 1,3-dioxacyclohexa-2,2-diyl diradical, preferably the 1,3-dioxacyclopenta-2,2-diyl diradical.
Suitably, $R^8$ is ethyl or butyl.
Suitably, $R^9$ is ethyl or butyl.
Preferably $R^6$ and $R^7$ are both methyl.
Preferably, $R^8$ is n-butyl.
Preferably, $R^9$ is n-butyl.

Preferred compounds of formula (III) are those selected from the list consisting of:
1,3-di-n-butylxanthin-7-yl propan-2-one, dimethyl ketal; and
2-methyl-2-[(1,3-di-n-butylxanthin-7-yl)methyl]-1,3-dioxolane.

Suitable compounds of formula (IV) are those exemplified in European Patent Specification No. 0017478.

Suitably, $R^{10}$ or $R^{11}$ is a methyl, ethyl, propyl or butyl group.

Suitably, $R^{10}$ and $R^{11}$ are linked so that the $C(OR^{10})(OR^{11})$ moiety is a 1,3-dioxacyclopenta-2,2-diyl or 1,3-dioxacyclohexa-2,2-diyl diradical.

Preferably, $R^{10}$ and $R^{11}$ are linked so that the $C(OR^{10})(OR^{11})$ moiety is a 1,3-dioxacyclopenta-2,2-diyl diradical.

A preferred compound of formula (IV) is 2-methyl-2-(theobrominyl-butyl-4)-1,3-dioxolane.

Suitable compounds of formula (V) are those exemplified in European Published Application No. 0042706. Suitably, $R^{12}$ is methyl, ethyl, n-propyl, iso-propyl or n-butyl.

Favourably, $R^{12}$ methyl and n-butyl, preferably n-butyl.

A preferred compound of formula (V) is 1-(5-oxohexyl)-3-n-butyl-7-(2-oxopropyl) xanthine.

An amount effective to treat proliferative skin disease depends on the usual factors such as the nature and severity of the problem and the weight of the human or non-human mammal. However, a unit dose will normally contain 5 to 500 mg for example 20 to 200 mg, of compound (I), (II), (III), (IV) or (V). Unit doses will normally be administered once or more than once a day, for example 2, 3, 4, 5 or 6 times a day, more usually 2 to 4 times a day, such that the total daily dose is normally in the range, for a 70 kg adult of 5 to 1000 mg, for example 50 to 600 mg, that is in the range of approximately 0.07 to 15 mg/kg/day, more usually 1 to 10 mg/kg/day, for example 2 to 5 mg/kg/day.

Suitably a compound of formula (I), (II), (III), (IV) or (V) is administered in the form of a unit-dose composition, such as a unit dose oral or parenteral composition.

Such compositions are prepared by admixture and are suitably adapted for oral or parenteral administration, and as such may be in the form of tablets, capsules, oral liquid preparations, powders, granules, lozenges, reconstitutable powders, injectable and infusable solutions or suspensions. Orally administrable compositions are preferred, in particular shaped oral compositions, since they are more convenient for general use.

Tablets and capsules for oral administration are usually presented in a unit dose, and contain conventional excipients such as binding agents, fillers, diluents, tabletting agents, lubricants, disintegrants, colourants, flavourings, and wetting agents. The tablets may be coated according to well known methods in the art.

Suitable fillers for use include cellulose, mannitol, lactose and other similar agents. Suitable disintegrants include starch, polyvinylpyrrolidone and starch derivatives such as sodium starch glycollate. Suitable lubricants include, for example, magnesium stearate. Suitable pharmaceutically acceptable wetting agents include sodium lauryl sulphate.
These solid oral compositions may be prepared by conventional methods of blending, filling, tabletting or the like. Repeated blending operations may be used to distribute the active agent throughout those compositions employing large quantities of fillers. Such operations are, of course, conventional in the art.

Oral liquid preparations may be in the form of, for example, aqueous or oily suspensions, solutions, emulsions, syrups, or elixirs, or may be presented as a

dry product for reconstitution with water or other suitable vehicle before use. Such liquid preparations may contain conventional additives such as suspending agents, for example sorbitol, syrup, methyl cellulose, gelatin, hydroxyethylcellulose, carboxymethyl cellulose, aluminium stearate gel or hydrogenated edible fats, emulsifying agents, for example lecithin, sorbitan monooleate, or acacia; non-aqueous vehicles (which may include edible oils), for example, almond oil, fractionated coconut oil, oily esters such as esters of glycerine, propylene glycol, or ethyl alcohol; preservatives, for example methyl or propyl p-hydroxybenzoate or sorbic acid, and if desired conventional flavouring or colouring agents.

Oral formulations also include conventional sustained release formulations, such as tablets or granules having an enteric coating.

For parenteral administration, fluid unit dose forms are prepared containing a compound of formula (I), (II), (III), (IV) or (V) and a sterile vehicle. The compound, depending on the vehicle and the concentration, can be either suspended or dissolved. Parenteral solutions are normally prepared by dissolving the compound in a vehicle and filter sterilising before filling into a suitable vial or ampoule and sealing. Advantageously, adjuvants such as a local anaesthetic, preservatives and buffering agents are also dissolved in the vehicle. To enhance the stability, the composition can be frozen after filling into the vial and the water removed under vacuum.

Parenteral suspensions are prepared in substantially the same manner except that the compound is suspended in the vehicle instead of being dissolved and

sterilised by exposure to ethylene oxide before suspending in the sterile vehicle. Advantageously, a surfactant or wetting agent is included in the composition to facilitate uniform distribution of the compound.

Compounds of formulae (I), (II), (III), (IV) or (V) may also be administered as a topical formulation in combination with conventional topical excipients.

Topical formulations may be presented as, for instance, ointments, creams or lotions, impregnated dressings gels, gel sticks, spray and aerosols, and may contain appropriate conventional additives such as preservatives, solvents to assist drug penetration and emollients in ointments and creams. The formulations may contain compatible conventional carriers, such as cream or ointment bases and ethanol or oleyl alcohol for lotions.

Suitable cream, lotion, gel, stick, ointment, spray, or aerosol formulations that may be used for compounds of formula (I), (II), (III), (IV) or (V) are conventional formulations well known in the art, for example, as described in standard text books of pharmaceutics and cosmetics, such as Harry's Cosmeticology published by Leonard Hill Books, Remington's Pharmaceutical Sciences, and the British and US Pharmacopoeias.

Suitably, the compound of formula (I), (II), (III), (IV) or (V) will comprise from about 0.5 to 20% by weight of the formulation, favourably from about 1 to 10% for example 2 to 5%.

No adverse toxicological effects are indicated in any of the above mentioned dosage ranges.

The following pharmacological data illustrates the activity of a compounds of formulae (I), (II), (III), (IV) or (V) in tests which are indicative of its potential use in the treatment of proliferative skin disease such as psoriasis.

## Pharmacological Data

The activity of cyclic AMP phosphodierterase in homogenates of rat gastocnemius muscle was measured in the presence of various concentrations of compounds of formulae (I), (II), (III), (IV) or (V) using 0.31 µM cyclic AMP as substration. The reciprocal of the reaction rate was plotted on the y axis against the concentration of each compound on the x axis (Dixon plot). The apparent Ki value was estimated by extrapolating the steeper linear portion of the Dixon plot to the x axis and obtaining the negative intercept on the x axis.

### Compounds of formula (I)

| $R^1$ | $R^2$ | X | Apparent Ki value. (µ Molar) |
|---|---|---|---|
| $C_4H_9$ | $C_4H_9$ | $-CH_2-$ | 3.3 |
| $C_4H_9$ | $C_4H_9$ | $-(CH_2)_4-$ | 14.1 |
| $C_4H_9$ | $C_4H_9$ | $-CH(CH_3)CH_2-$ | 17.5 |
| $C_4H_9$ | $C_2H_5$ | $-CH(CH_3)-$ | 24.8 |
| $C_4H_9$ | $C_4H_9$ | $-CH(CH_3)-$ | 30.1 |
| $C_4H_9$ | $C_4H_9$ | $-(CH_2)_3-$ | 39.0 |
| $C_4H_9$ | $C_2H_5$ | $-CH_2-$ | 21 |
| $C_4H_9$ | $C_3H_7$ | $-CH_2-$ | 2.0 |
| $i-C_3H_7$ | $i-C_3H_7$ | $-CH_2-$ | 6.1 |
| $C_4H_9$ | $C_2H_5$ | $-(CH_2)_2-$ | 12 |
| $C_4H_9$ | $C_4H_9$ | $-(CH_2)_2-$ | 25 |
| $C_3H_7$ | $C_3H_7$ | $-(CH_2)_2-$ | 27 |

Compounds of formula (II)

| $R^4$ | $R^5$ | $X^2$ | Y | m | Apparent Ki Value (μ Molar) |
|---|---|---|---|---|---|
| $C_4H_9$ | $C_4H_9$ | S | 0 | 1 | 2.5 |
| $C_4H_9$ | $C_2H_5$ | 0 | S | 2 | 22.9 |
| $C_4H_9$ | $C_2H_5$ | 0 | S | 1 | 41 |

Compounds of formula (III)

| $R^6$ | $R^7$ | $R^8$ | $R^9$ | Apparent Ki value (μ Molar) |
|---|---|---|---|---|
| $CH_2$ | $CH_2$ | $C_4H_9$ | $C_4H_9$ | 29.0 |
| $CH_2$ | $CH_2$ | $C_4H_9$ | $C_2H_5$ | 45 |
| $CH_3$ | $CH_3$ | $C_4H_9$ | $C_4H_9$ | 27 |
| $CH_2$ | $CH_2$ | $C_2H_5$ | $C_2H_5$ | 73 |

Compounds of formula (V).

| $R^{12}$ | Apparent Ki value (µ Molar) |
|---|---|
| $C_4H_9$ | 3.9 |

0195496

Claims

1. The use of a compound of formula (I):

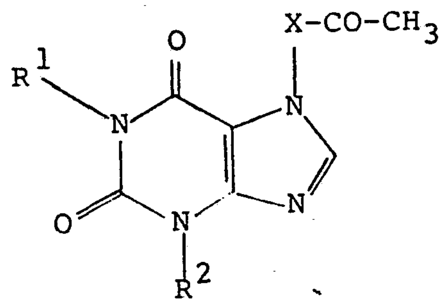

(I)

wherein $R^1$ and $R^2$, which may be the same or different, each represents a straight-chain or branched-chain alkylradical of 2 to 6 carbon atoms, or a cyclohexyl, alkoxyalkyl or hydroxyalkyl radical, and X represents a hydrocarbon radical having up to 4 carbon atoms which may be substituted by a methyl group;

a compound of formula (II):

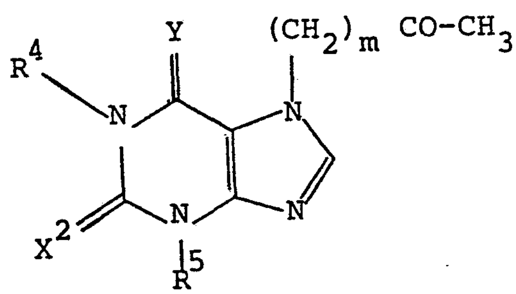

(II)

wherein

$X^2$ is sulphur and Y is oxygen or sulphur:

$R^4$ is an alkyl group of up to 6 carbon atoms;

$R^5$ is an alkyl group of up to 6 carbon atoms; and m is 1; or

$X^2$ is oxygen and Y is sulphur; one of $R^4$ and $R^5$ is an alkyl group of up to 6 carbon atoms and the other is an alkyl group of 2 to 3 carbon atoms; and m is 1 or 2;

a compound of formula (III):

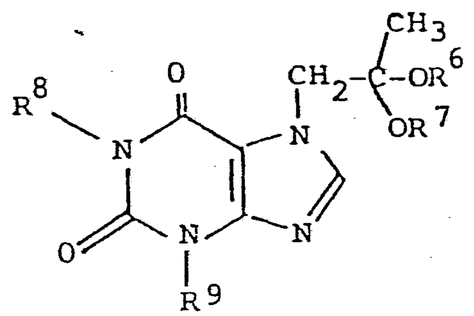

(III)

wherein

$R^6$ is a lower alkyl group and $R^7$ is a lower alkyl group; or $R^6$ is linked to $R^7$ so that the $OR^6$ and $OR^7$ moieties and the carbon atom to which they are attached form a 1,3-dioxacyclohexa -2,2-diyl, 1,3-dioxa-cyclopenta -2,2-diyl or 1,3-dioxacyclohepta-2,2-diyl diradiacal; and $R^8$ and $R^9$ are the same or different and are each a lower alkyl group:

a compound of formula (IV):

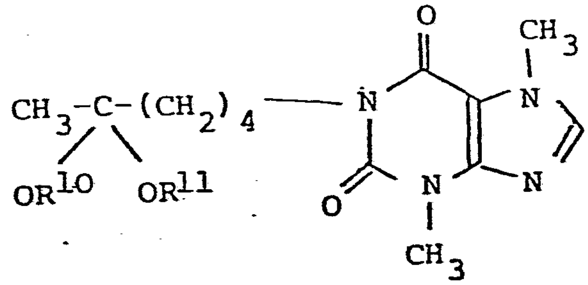

(IV)

wherein $R^{10}$ is a lower alkyl;
group and $R^{11}$ is a lower alkyl group optionally linked to $R^{10}$ so that the $OR^{10}$ and $OR^{11}$ moieties and the carbon atom to which they are attached form a 1,3-dioxacyclohexa-2,2-diyl, 1,3-di oxacyclo-penta-2,2-diyl or 1,3-dioxacyclohepta-2,2-diyl diradical; or

a compound of formula (V):

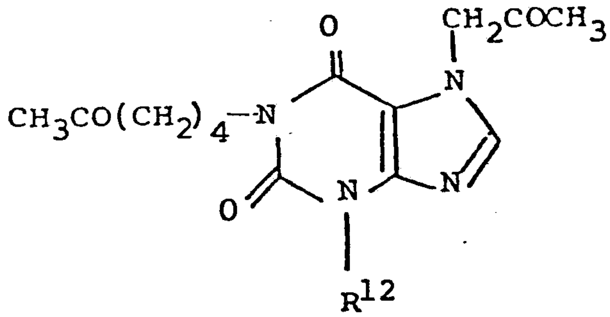

(V)

wherein $R^{12}$ is $C_{1-4}$ alkyl; for the manufacture of a medicament for treating proliferate skin disease in human or non-human mammals.

2. A use as claimed in claim 1, wherein $R_1$ is n-butyl.

3. A use as claimed in claim 1 or claim 2, wherein $R^2$ is n-butyl.

4. A use as claimed in claim 1 or claim 2, wherein X is $-CH_2-$.

5. A use as claimed in claim 1, wherein $R^4$ is n-butyl.

6. A use as claimed in claim 1 or claim 5, wherein $R^5$ is n-butyl.

7. A use as claimed in claims 1, 5 or 6, wherein $X^2$ is sulphur and Y is oxygen.

8. A use as claimed in claim 1, wherein $R^{12}$ is n-butyl.

9. A use as claimed in claim 1, wherein the compound of formula (I) is 1,3-di-n-butyl-7-(2-oxopropyl)-xanthine.

10. A use as claimed in claim 1, wherein the compound of formula (II) is 1,3-di-n-butyl-7-(2-oxopropyl)-2-thioxanthine.

11. A use as claimed in claim 1, wherein the compound of formula (V) is 1-(5-oxohexyl)-3-n-butyl-7-(2-oxopropyl)xanthine.